# EUROPÄISCHE PATENTSCHRIFT

(11) **EP 1 978 969 B1**
(45) Veröffentlichungstag und Bekanntmachung des Hinweises auf die Patenterteilung: **18.07.2012**
(21) Anmeldenummer: 07702961.9
(22) Anmeldetag: 23.01.2007
(51) Int. Cl.: A61K 31/565, A61K 31/57

(54) **HORMONKOMBINATION ZUR LINDERUNG VON MENSTRUATIONSZUKLUSABHÄNGIGEN STIMMUNGSSCHWANKUNGEN**
HORMONE COMBINATION FOR ALLEVIATION OF MENSTRUAL CYCLE DEPENDENT MOOD FLUCTUATIONS
ASSOCIATION D'HORMONES POUR SOULAGER LES VARIATIONS D'HUMEUR DUES AUX MENSTRUATIONS

(30) Priorität: 24.01.2006 DE 102006003508
(43) Veröffentlichungstag der Anmeldung: 15.10.2008
(73) Patentinhaber: Richter Gedeon Nyrt., 1103 Budapest (HU)
(72) Erfinder: SCHRAMM, Georg, 52224 Stolberg (DE); KNEIP, Christa, 52080 Aachen (DE)
(74) Vertreter: Kutzenberger, Helga
(86) Internationale Anmeldenummer: PCT/EP2007/000552
(87) Internationale Veröffentlichungsnummer: WO 2007/085420

(56) Entgegenhaltungen:
- DE-A1- 4 321 957
- DE-A1- 4 339 934
- BUNDESVERBAND DER PHARMAZEUTISCHEN INDUSTRIE E.V UND WEITERE: "Rote Liste 2002" 2002, ROTE LISTE SERVICE GMBH, FRANKFURT/MAIN , GERMANY , XP002432757 siehe "Belara" N.: 76131
- HONMA S ET AL: "IDENTIFICATION AND ANTI-ANDROGENIC ACTIVITY OF THE METABOLITES OF 17ALPHA-ACETOXY-6-CHLOROPREGNA-4,6-DIENE-3 ,20-DIONE (CHLORMADINONE ACETATE) IN THE RAT, RABBIT, DOG AN DMAN" CHEMICAL AND PHARMACEUTICAL BULLETIN, PHARMACEUTICAL SOCIETY OF JAPAN, TOKYO, JP, Bd. 25, Nr. 8, 25. August 1977 (1977-08-25), Seiten 2019-2031, XP009083454 ISSN: 0009-2363

## Beschreibung

Die vorliegende Erfindung betrifft die Verwendung einer Hormonkombination aus wenigstens einem Östrogen ausgewählt aus der Gruppe bestehend aus Ethinylestradiol (I) und Östradiol (II) als Östrogenkomponente und wenigstens einem Metaboliten des Chlormadinonacetats ausgewählt aus der Gruppe bestehend aus 3α-Hydroxy-6-chlor-17α-acetoxy-4,6-pregnadien-20-on (3α-Hydroxy-chlormadinonacetat) und 3β-Hydroxy-6-chlor-17α-acetoxy-4,6-pregnadien-20-on (3β-Hydroxy-chlormadinonacetat), (Honma, S. et al., Chem. Pharm. Bull., 25 (8) 2019-2031, 1977), gegebenenfalls gemischt mit Chlormadinonacetat als Gestagenkomponente zur Herstellung eines Arzneimittels zur Linderung von menstruationszyklusabhängigen Stimmungsschwankungen bei Frauen.

Viele Frauen leiden während ihres Menstruationszyklus unter Stimmungsschwankungen, die auch auftreten können, selbst wenn keine äußeren, erkennbaren Einflüsse, Beschwerden und/oder Störungen und/oder keine Beschwerden und/oder Störungen verbunden mit dem Menstruationszyklus einer Frau vorliegen. Diese Stimmungsschwankungen können sich nicht nur in einer erhöhten Gereiztheit in bestimmten Phasen des Menstruationszyklus, sondern auch in Niedergeschlagenheit und/oder erhöhter Empfindlichkeit bis hin zur Trauer ausdrücken, ohne dass es dafür eine erkennbare Erklärung gibt.

Es versteht sich von selbst, dass solche Stimmungsschwankungen, die von vielen Frauen als psychische Beeinträchtigung empfunden werden, die Lebensqualität von Frauen beeinträchtigt. Hinzu kommt noch, dass diese Stimmungsschwankungen regelmäßig mit Ablauf des Menstruationszyklus immer wieder auftreten, so dass viele Frauen das Bedürfnis haben, solchen Stimmungsschwankungen nicht immer wieder ausgesetzt sein zu müssen bzw. diese Stimmungsschwankungen zumindest im Ablauf eines Menstruationszyklus lindern und/oder sogar die Stimmung insgesamt verbessern zu können.

Es besteht daher ein Bedarf, ein Arzneimittel für Frauen zur Verfügung zu stellen, das zumindest zu einer Linderung, vorzugsweise zur Verhinderung, von menstruationszyklusabhängigen Stimmungsschwankungen, die üblicherweise ohne erkennbaren (weiteren) Grund auftreten, bis hin zu einer insgesamten Gemütsaufhellung während des gesamten Menstruationszyklus geeignet ist.

Diese Aufgabe wird durch die Verwendung einer Hormonkombination aus wenigstens einem Östrogen ausgewählt aus der Gruppe bestehend aus Ethinylestradiol (I) und Östradiol (II) als Östrogenkomponente und wenigstens einem Metaboliten des Chlormadinonacetats ausgewählt aus der Gruppe bestehend aus 3α-Hydroxy-6-chlor-17α-acetoxy-4,6-pregnadien-20-on (3α-Hydroxy-chlormadinonacetat) und 3β-Hydroxy-6-chlor-17α-acetoxy-4,6-pregnadien-20-on (3β-Hydroxy-chlormadinonacetat), , gegebenenfalls gemischt mit Chlormadinonacetat als Gestagenkomponente zur Herstellung eines Arzneimittels zur Linderung von menstruationszyklusabhängigen Stimmungsschwankungen, vorzugsweise bis hin zur Gemütsaufhellung bei Frauen, gelöst.

Das Arzneimittel wird vorzugsweise in Form von Tages-Einheiten bereitgestellt. Vorzugsweise wird zur Herstellung einer Tages-Einheit eine Hormonkombination bestehend aus vorzugsweise 0,001 bis 50 µg, besonders bevorzugt aus 5 bis 50 µg, ganz besonders bevorzugt aus 5 bis 30 µg, der Östrogenkomponente (I) oder vorzugsweise 0,5 bis 4 mg, ganz besonders bevorzugt 0,5 bis 2 mg, ganz besonders bevorzugt aus 1 mg, der Östrogenkomponente (II) und vorzugsweise 1 bis 10 mg, besonders bevorzugt 1 bis 5 mg, ganz besonders bevorzugt ≥ 2 mg der Gestagenkomponente und gegebenenfalls übliche Hilfsstoffe eingesetzt.

Erfindungsgemäß kann als Gestagenkomponente eine der folgenden Komponenten
a) bis c) oder h),
   a) 3α-Hydroxy-chlormadinonacetat oder
   b) 3β-Hydroxy-chlormadinonacetat oder
   c) eine Mischung von a) und b) in einem beliebigen Mischungsverhältnis oder
   h) eine Mischung von Chlormadinonacetat (CMA) mit a) und/oder b) in einem Mischungsverhältnis von 10 bis 90 Gew.% CMA und 90 bis 10 Gew.% von a) und/oder b), bezogen auf die Gesamtmischung,
eingesetzt werden.

Sofern auch ein ausreichender kontrazeptiver Schutz bei den Frauen erzielt werden soll, sollte zur Herstellung einer Tages-Einheit eine Hormonkombination bestehend aus jeweils mindestens 15 µg, vorzugsweise 20 µg oder 30 µg Ethinylestradiol und/oder mindestens 0,5 mg, vorzugsweise 1 mg oder 2 mg Östradiol und jeweils wenigstens 2, vorzugsweise 2, 3, 4 oder 5 mg einer der Gestagenkomponenten a) bis c) oder h) und gegebenenfalls üblichen Hilfsstoffen eingesetzt werden.

Ganz besonders bevorzugt eignet sich auch zur Erzielung einer kontrazeptiven Wirkung der Einsatz einer Hormonkombination aus jeweils 20 µg Ethinylestradiol oder 1 mg Östradiol und ≥ 2 mg der Gestagenkomponenten a) bis c) oder h)

Das erfindungsgemäß zum Einsatz kommende Arzneimittel wird vorzugsweise in Form von Tabletten formuliert, die neben der aufgeführten Hormonkombination gegebenenfalls noch übliche Hilfsstoffe enthalten. Diese Tabletten werden insbesondere in Form von mindestens 21, vorzugsweise 21 bis 25, die Hormonkombination enthaltenden Tages-Einheiten, die für eine ununterbrochene, orale Einnahme gedacht sind, gefolgt von einer 3 bis 7-tägigen Einnahmepause oder in Kombination mit 7 bis 3 hormonfreien Tages-Einheiten für eine ununterbrochene, orale Einnahme bereitgestellt.

Zur Linderung bzw. Vermeidung von menstruationszyklusabhängigen Stimmungsschwankungen neben einer gegebenenfalls hormonalen Kontrazeption kann das Arzneimittel in Form von die Hormonkombination enthaltenden Tages-Einheiten auch für eine ununterbrochene Verabreichung über mehrere Jahre, vorzugsweise bis zu 2 Jahren, besonders bevorzugt bis zu 1 Jahr, gegebenenfalls in Kombination mit 7 bis 3 hormonfreien Tages-Einheiten zur ununterbrochenen Verabreichung oder gefolgt von einer 7 bis 3-tägigen Einnahmepause zur Verfügung gestellt werden.

Das erfindungsgemäß zubereitete Arzneimittel kann aber auch für die erfindungsgemäße Verwendung in einer Darreichungsform mit weniger als 365 die Hormonkombination enthaltenden Tages-Einheiten, wie z. B. mit 77 bis 193 bzw. 42 bis 52, die Hormonkombination enthaltenden Tages-Einheiten zur ununterbrochenen oralen Verabreichung, gefolgt von einer Einnahmepause über 7 bis 3 Tage oder in Kombination mit 7 bis 3 hormonfreien Tages-Einheiten zur ununterbrochenen Verabreichung bereitgestellt werden.

Wie bereits dargelegt, kann anstelle der 7 bis 3 hormonfreien Tages-Einheiten auch eine entsprechend lange Einnahmepause gemacht werden. Dementsprechend kann die orale Darreichungsform mit den vorstehend aufgeführten Zahl an die Hormonkombination enthaltenden Tages-Einheiten auch als Kit vorliegen, das mehrere dieser Darreichungsformen zur fortgeführten Einnahme, unterbrochen von einer entsprechenden Einnahmepause umfasst. Selbstverständlich kann ein Kit auch mehrere orale Darreichungsformen umfassen, die eine ununterbrochene Einnahme von die Hormonkombination enthaltenden Tages-Einheiten in Kombination für eine ununterbrochene Einnahme der aufgeführten Zahl von hormonfreien Tages-Einheiten aufweist.

Vorzugsweise weist jede der die Hormonkombination enthaltenden Tages-Einheiten dieselbe Menge der Östrogenkomponente bzw. der Gestagenkomponente auf, d. h. sowohl die Menge an Ethinylestradiol und/oder Östradiol als auch an einer der Gestagenkomponenten a) bis c) oder h) wird über einen Einnahmezyklus, der wie vorstehend ausgeführt bis zu mehreren Jahren dauern kann, konstant gehalten.

Durch die Verwendung insbesondere eines monophasigen Arzneimittels gelingt es erfindungsgemäß, menstruationszyklusabhängige Stimmungsschwankungen nicht nur zu lindern, sondern zu verhindern, wobei nicht nur eine Verschlechterung des Gemütszustandes bis hin zu einem seelischen Tiefstand bei Frauen verhindert wird, die unter menstruationszyklusabhängigen Stimmungsschwankungen leiden, sondern auch das seelische Befinden, d. h. den Gemütszustand einer Frau während ihres gesamten Menstruationszyklus so zu verbessern, dass die Stimmung insgesamt dabei aufgehellt wird.

Das zum Einsatz kommende Arzneimittel liegt vorzugsweise als orale Darreichungsform, ganz besonders bevorzugt in Form von Tabletten, vor. Dabei entsprechen Tages-Einheiten jeweils einer Tablette, die entsprechend einen Einnahmezyklus, vorzugsweise in Blistern verpackt, vorzugsweise unter Kennzeichnung der jeweils einzunehmenden Tages-Einheit, und als Arzneimittelpackung enthaltend mindestens einen solchen Blister, vorzugswiese mindestens 3 Blister für die jeweilige Anzahl von Einnahmezyklen bzw. für eine angestrebte ununterbrochene Verabreichung marktgeführt werden.

### In vitro-Daten

Es ist bekannt, dass das endogen gebildete Neurosteroid Allopregnanolon einen positiven Effekt auf die Stimmung hat. Plasmakonzentrationen von Allopregnanolon sind in Patientinnen mit depressiver Stimmung vermindert. Die positive Wirkung des Allopregnanolons auf die Stimmung wird auf dessen Wechselwirkung mit GABA_{A}-Rezeptoren zurückgeführt. An diesem Rezeptor des Zentralnervensystems wirkt Allopregnanolon als positiver allosterischer Modulator und führt damit zu anxiolytischen und stimmungsaufhellenden Effekten.

Es wurde gefunden, dass 3-α-OH-CMA bzw. 3-β-OH-CMA Bindungseigenschaften an GABA_{A}-Rezeptoren zeigen, die denen des Allopregnanolons sehr ähnlich sind. Allopregnanolon beeinflusst die Bindung von radioaktiv markiertem Muscimol (Agonist) an diese Rezeptoren.

### 1) Einfluss von Allopregnanolon, 3-α-OH-CMA bzw. 3-β-OH-CMA auf die Bindung von Muscimol an GABA_{A}-Rezeptoren (Rattenhirn)

Die nachfolgenden Bestimmungen des Einflusses von Allopregnanolon, 3-α-OH-CMA bzw. 3-β-OH-CMA auf die Bindung von Muscimol an GABA_{A}-Rezeptoren (Ratte) basieren auf der Veröffentlichung der experimentellen Vorschriften von Snodgrass, S.R. (Nature, 1979, V273, p.392-394). Die entsprechende Offenbarung wird hiermit als Referenz eingeführt und ist Teil der Offenbarung der vorliegenden Anmeldung.

### A) Allopregnanolon

Membranpräparationen aus cerebralem Rattencortexmaterial werden hergestellt. Als Referenzsubstanz wird 5 nM [³H] Muscimol, zur Bestimmung der unspezifischen Bindung wird 10 µM Muscimol eingesetzt. Die Inkubationsdauer der Substanzen ([³H] Muscimol, Allopregnanolon) am Rezeptor beträgt jeweils 10 Minuten bei einer Temperatur von ca. 4°Celsius. Die Stammlösung (5 x 10⁻² M) von Allopregnanolon wird jeweils in 75%-igem DMSO 1:10 vorverdünnt und anschließend 1:5 in 25%-igem DMSO weiterverdünnt. Die finale Konzentration von Allopregnanolon im Test ist jeweils 1x10⁻¹⁰M, 1x10⁻⁹M, 1x10⁻⁸M, 1x10⁻⁷ M, 1x10⁻⁶ M und 1x10⁻⁵ M. Nach der vorstehend genannten Inkubationsdauer der Substanzen werden die Inkubationsansätze nach Standardvorschriften filtriert, gewaschen und die Radioaktivität der Filter mit einem Scintillationsmeßgerät bestimmt. Derartige Standardvorschriften sind dem Fachmann bekannt. Die Experimente werden jeweils in Doppelansätzen durchgeführt.

Es kann gezeigt werden, dass bei einer Konzentration von 1 µM Allopregnanolon die Bindung des radioaktiv markierten Muscimols (5 nM) um 38% erhöht wird.

### B) 3-α-OH-CMA bzw. 3-β-OH-CMA

Die Bestimmung des Einflusses von 3-α-OH-CMA bzw. 3-β-OH-CMA auf die Bindung von Muscimol an GABA_{A}-Rezeptoren (Ratte) erfolgt analog zu der vorstehend beschriebenen Methode. Statt Allopregnanolon wird 3-α-OH-CMA bzw. 3-β-OH-CMA verwendet.

Es kann gezeigt werden, dass bei einer Konzentration von 0,1 µM 3-β-OH-CMA die Bindung des radioaktiv markierten Muscimols (5 nM) um 31 % und bei einer Konzentration von 0,001 µM 3-α-OH-CMA um 12% erhöht wird.

Daraus kann auf einen stimmungsaufhellenden Effekt von 3-α-OH-CMA bzw. 3-β-OH-CMA geschlossen werden.

### 2) Bestimmung der Affinität von 3-α-OH-CMA bzw. 3-β-OH-CMA zum humanen Progesteronrezeptor

Die nachfolgende Bestimmung der Affinität von 3-α-OH-CMA bzw. 3-β-OH-CMA zum humanen Progesteronrezeptor basiert auf der Veröffentlichung der experimentellen Vorschriften von Eckert et al. (Cancer Research, 1982, V42, p.139-144). Die entsprechende Offenbarung wird hiermit als Referenz eingeführt und ist Teil der Offenbarung der vorliegenden Anmeldung.

Die cytosolischen Fraktionen von MCF-7-Zellen, welche den humanen Progesteronrezeptor enthalten, werden verwendet. Als Referenzsubstanz wird 2 nM [³H] R 5020, zur Bestimmung der unspezifischen Bindung wird 1 µM R 5020 eingesetzt. Die Inkubationsdauer der Substanzen ([³H] R 5020, 3-α-OH-CMA bzw. 3-β-OH-CMA) am Rezeptor beträgt jeweils 20 Stunden bei einer Temperatur von ca. 4°Celsius. Stammlösungen (5 x 10⁻² M) von 3-α-OH-CMA bzw. 3-β-OH-CMA werden jeweils in 75%-igem DMSO 1:10 vorverdünnt und anschließend 1:5 in 25%-igem DMSO weiterverdünnt. Die finalen Konzentrationen von 3-α-OH-CMA bzw. 3-β-OH-CMA im Test sind 3x10⁻¹⁰ M, 3x10⁻⁹ M, 1x10⁻⁸ M, 3x10⁻⁸ M, 1x10⁻⁷ M, 3x10⁻⁷ M, 1x10⁻⁶ M und 1x10⁻⁵ M. Nach der vorstehend genannten Inkubationsdauer der Substanzen werden die Inkubationsansätze nach Standardvorschriften filtriert, gewaschen und die Radioaktivität der Filter mit einem Scintillationsmeßgerät bestimmt. Derartige Standardvorschriften sind dem Fachmann bekannt. Die Experimente werden jeweils in Doppelansätzen durchgeführt.

Die entsprechenden IC₅₀-Werte werden über eine nicht-lineare Regressionsanalyse der Verdrängungskurven über die Hill-Kurvenangleichungsformel berechnet. Ein derartiges Berechnungsverfahren ist dem Fachmann bekannt.

Die entsprechenden Kᵢ-Werte (Inhibitionskonstante) werden über die Cheng-Prusoff-Gleichung bestimmt (Kᵢ = IC₅₀/(1+(L/K_{D})), wobei L der Konzentration des Radioliganden im Test und K_{D} der Affinität des Radioliganden zum Rezeptor entspricht).

Die Messwerte sind in der nachfolgenden Tabelle zusammengefasst.

### 3) Bestimmung der Affinität von 3-α-OH-CMA bzw. 3-β-OH-CMA zum humanen Androgenrezeptor

Die nachfolgende Bestimmung der Affinität von 3-α-OH-CMA bzw. 3-β-OH-CMA zum humanen Androgenrezeptor basiert auf der Veröffentlichung der experimentellen Vorschriften von Zava et al. (Endocrinology, 1979, V104, p.1007-1012). Die entsprechende Offenbarung wird hiermit als Referenz eingeführt und ist Teil der Offenbarung der vorliegenden Anmeldung.

Die cytosolischen Fraktionen von LNCaP-Zellen, welche den humanen Androgenrezeptor enthalten, werden verwendet. Als Referenzsubstanz wird 0.5 nM [³H] Methyltrienolon, zur Bestimmung der unspezifischen Bindung wird 1 µM Miboleron eingesetzt. Die Inkubationsdauer der Substanzen ([³H] Methyltrienolon, 3-α-OH-CMA bzw. 3-β-OH-CMA) am Rezeptor beträgt jeweils 24 Stunden bei einer Temperatur von ca. 4°Celsius. Die Stammlösungen, die Verdünnungsreihen von 3-α-OH-CMA bzw. 3-β-OH-CMA, die Waschschritte, die Bestimmung der Radioaktivität, sowie die Methoden zur Berechnungen der jeweiligen IC₅₀ und Kᵢ-Werte entsprechen dem unter Punkt 2) beschriebenen experimentellen Protokoll.

Die Messwerte sind in der nachfolgenden Tabelle zusammengefasst.

| **Progesteronrezeptor (human)** | | |
|---|---|---|
| **Substanz** | IC₅₀ [nM] | Kᵢ [nM] |
| 3-α-OH-CMA | 39 | 13 |
| 3-β-OH-CMA | 18 | 6 |

| **Androgenrezeptor (human)** | | |
|---|---|---|
| **Substanz** | **IC₅₀** [nM] | **Kᵢ** [nM] |
| 3-α-OH-CMA | 100 | 83 |
| 3-β-OH-CMA | 25 | 20 |

Aus den Daten geht hervor, dass sowohl 3-α-OH-CMA als auch 3-β-OH-CMA eine hohe Affinität zum humanen Progesteronrezeptor und zum humanen Androgenrezeptor haben. Daraus ist deren jeweilige kontrazeptive und anti-androgene Wirkung ableitbar.

### Beispiele

### a) Herstellung des Arzneimittels

### Beispiel 1:

### Zusammensetzung

| | Pro Tablette | Pro Charge |
|---|---|---|
| Ethinylestradiol | 0,020 mg | 0,0020 kg |
| 3α-Hydroxychlormadinonacetat | 3,000 mg | 0,3000 kg |
| 3β-Hyrdoxychlormadinonacetat | 2,000 mg | 0,2000 kg |
| Povidon K30 | 3,000 mg | 0,3000 kg |
| Lactose | 31,980 mg | 3,1980 kg |
| Maisstärke | 0,500 mg | 1,0500 kg |
| Hochdisperses Siliciumdioxid | 0,500 mg | 0,0500 kg |

Ethinylestradiol (EE) und Povidone K 30 (PVP) wurden in 600 ml Ethanol gelöst. 3α-Hydroxychlormadinonacetat und 3β-Hydroxychformadinonacetat (Partikelgröße 90% <50µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit hochdispersem Siliciumdioxid gemischt und auf einer Tablettenpresse mit 5 mm Stempeln zu Tabletten mit einem Gewicht von 50 mg gepresst.

Die Tabletten wurden mit einem Lack auf Basis Methylhydroxypropylcellulose mit folgender Zusammensetzung überzogen (Überzugsmasse 2 mg pro Tablette)

| | |
|---|---|
| Methylhydroxypropylcellulose 6 mPa · s, | 0,1351 kg |
| Polyethylenglykol 6000 | 0,0395 kg |
| Propylenglykol | 0,0054 kg |
| Gereinigtes Wasser | 1,6200 kg |

Jeweils 24 überzogene Tabletten wurden als die Hormonkombination enthaltenden Tages-Einheiten und jeweils 4 entsprechend zusammengesetzte hormonfreie, überzogene Tabletten in einem Blister verpackt.

## Patentansprüche

1. Verwendung einer Hormonkombination aus wenigstens einem Östrogen usgewählt aus Ethinylestradiol (I) oder Östradiol (II) als Östrogenkomponente und wenigstens einem Metaboliten des Chlormadinonacetats ausgewählt aus 3α-Hydroxy-6-chlor-17α-acetoxy-4,6-pregnadien-20-on (3α-Hydroxy-chlormadinonacetat), oder 3β-Hydroxy-6-chlor-17α-acetoxy-4,6-pregnadien-20-on (3β-Hydroxy-chlormadinonacetat), gegebenenfalls gemischt mit Chlormadinonacetat als Gestagenkomponente zur Herstellung eines Arzneimittels zur Linderung von menstruationszyklusabhängigen Stimmungsschwankungen bei Frauen.

2. Verwendung gemäß Anspruch 1, **dadurch gekennzeichnet, dass** das Arzneimittel in Form von Tages-Einheiten bereitgestellt wird.

3. Verwendung gemäß Anspruch 1 oder 2, **dadurch gekennzeichnet, dass** zur Herstellung einer Tages-Einheit eine Hormonkombination bestehend aus 5 bis 50 µg der Östrogenkomponente (I) und/oder 0,5 bis 4 mg der Östrogenkomponente (II) und 1 bis 10 mg der Gestagenkomponente und gegebenenfalls übliche Hilfsstoffe eingesetzt werden.

4. Verwendung gemäß Anspruch 1 oder 2, **dadurch gekennzeichnet, dass** zur Herstellung einer Tages-Einheit eine Hormonkombination bestehend aus 5 bis 30 µg der Östrogenkomponente (I) und/oder 0,5 bis 2 mg der Östrogenkomponente (II) und 1 bis 10 mg der Gestagenkomponente und gegebenenfalls übliche Hilfsstoffe eingesetzt werden.

5. Verwendung gemäß einem der Ansprüche 1 bis 4, **dadurch gekennzeichnet, dass** als Gestagenkomponente einer der folgenden Komponenten a) bis c) oder h),
a) 3α-Hydroxy-chlormadinonacetat oder
b) 3β-Hydroxy-chlormadinonacetat oder
c) eine Mischung von a) und b) in einem beliebigen Mischungsverhältnis oder
h) eine Mischung von Chlormadinonacetat mit a) und/oder b) in einem Mischungsverhältnis von 10 bis 90 Gew.% CMA und 90 bis 10 Gew.% von a) und/oder b), bezogen auf die Gesamtmischung,
eingesetzt werden.

6. Verwendung gemäß Anspruch 1, **dadurch gekennzeichnet, dass** zur Herstellung einer Tages-Einheit eine Hormonkombination bestehend aus jeweils mindestens 15 µg, vorzugsweise 20 µg oder 30 µg Ethinylestradiol und/oder mindestens 0,5 mg, vorzugsweise 1 mg oder 2 mg Östradiol und jeweils mindestens 1, vorzugsweise 2, 3, 4 oder 5 mg einer der Gestagenkomponenten a) bis c) oder h) und gegebenenfalls üblichen Hilfsstoffen eingesetzt werden.

7. Verwendung gemäß Anspruch 6, **dadurch gekennzeichnet, dass** die Hormonkombination aus jeweils 20 µg oder 30 µg Ethinylestradiol und/oder 1 mg Östradiol und ≥ 2 mg der Gestagenkomponenten a) bis c) oder h) besteht.

8. Verwendung gemäß einem der Ansprüche 1 bis 7, **dadurch gekennzeichnet, dass** das Arzneimittel in Form von mindestens 21, die Hormonkombination enthaltenden Tages-Einheiten zur ununterbrochenen Verabreichung gegebenenfalls gefolgt von einer 7 bis 3-tägigen Einnahmepause oder von 7 bis 3 hormonfreien Tages-Einheiten zur ununterbrochenen Verabreichung bereitgestellt wird.

9. Verwendung gemäß Anspruch 8, **dadurch gekennzeichnet, dass** das Arzneimittel in Form von die Hormonkombination enthaltenden Tages-Einheiten für eine ununterbrochene Verabreichung über mehrere Jahre, vorzugsweise bis zu 2 Jahren, besonders bevorzugt bis zu 1 Jahr, und von 7 bis 3 hormonfreien Tages-Einheiten zur ununterbrochenen Verabreichung oder gefolgt von einer Einnahmepause von 3 bis 7 Tagen bereitgestellt wird.

10. Verwendung gemäß Anspruch 8, **dadurch gekennzeichnet, dass** das Arzneimittel in Form von 77 bis 193, die Hormonkombination enthaltenden Tages-Einheiten zur ununterbrochenen Verabreichung und von 7 bis 3 hormonfreien Tages-Einheiten zur ununterbrochenen Verabreichung oder gefolgt von einer Einnahmepause von 3 bis 7 Tagen bereitgestellt wird.

11. Verwendung gemäß Anspruch 8, **dadurch gekennzeichnet, dass** das Arzneimittel in Form von 42 bis 52, die Hormonkombination enthaltenden Tages-Einheiten zur ununterbrochenen Verabreichung und von 7 bis 3 hormonfreien Tages-Einheiten zur ununterbrochenen Verabreichung oder gefolgt von einer Einnahmepause von 3 bis 7 Tagen bereitgestellt wird.

12. Verwendung gemäß Anspruch 8, **dadurch gekennzeichnet, dass** das Arzneimittel in Form von 21 bis 25, die Hormonkombination enthaltenden Tages-Einheiten zur ununterbrochenen Verabreichung und von 7 bis 3 hormonfreien Tages-Einheiten zur ununterbrochenen Verabreichung oder gefolgt von einer 3 bis 7-tägigen Einnahmepause bereitgestellt wird.

13. Verwendung gemäß einem der Ansprüche 1 bis 12, **dadurch gekennzeichnet, dass** das Arzneimittel in jeder der die Hormonkombination enthaltenden Tages-Einheiten mengenmäßig dieselbe Kombination aus Ethinylestradiol oder Östradiol und eine Gestagenkomponenten a) bis c) oder h) aufweist.

14. Verwendung gemäß einem der Ansprüche 1 bis 13 zur Verhinderung von menstruationszyklusabhängigen Stimmungsschwankungen, vorzugsweise zur Verhinderung einer Verschlechterung des Gemütszustandes bei Frauen.

15. Verwendung gemäß einem der Ansprüche 1 bis 13 zum Ausgleich von menstruationszyklusabhängigen Stimmungsschwankungen.

16. Verwendung gemäß einem der Ansprüche 1 bis 13 zur Verbesserung des Gemütszustandes und Stimmungsaufhellung während eines gesamten weiblichen Menstruationszyklus.

17. Verwendung gemäß einem der Ansprüche 7 bis 13 zur Linderung von menstruationszyklusabhängigen Stimmungsschwankungen bei Frauen.

## Claims

1. Use of a hormone combination of at least one oestrogen selected from ethinyl estradiol (I) or estradiol (II) as oestrogen component and at least one metabolite of chlormadinone acetate selected from 3α-hydroxy-6-chloro-17α-acetoxy-4,6-pregnadien-20-one (3α-hydroxy-chlormadinone acetate) or 3β-hydroxy-6-chloro-17α-acetoxy-4,6-pregnadien-20-one (3β-hydroxy-chlormadinone acetate), optionally mixed with chlormadinone acetate as gestagen component for producing a medicinal drug for alleviating menstrual cycle-dependent mood swings in women.

2. Use according to claim 1, **characterised in that** the medicinal drug is provided in the form of daily units.

3. Use according to claim 1 or 2, **characterised in that** a daily unit is produced by using a hormone combination consisting of 5 to 50 µg of the oestrogen component (I) and/or 0.5 to 4 mg of the oestrogen component (II) and 1 to 10 mg of the gestagen component and optionally conventional auxiliary substances.

4. Use according to claim 1 or 2, **characterised in that** a daily unit is produced by using a hormone combination consisting of 5 to 30 µg of the oestrogen component (I) and/or 0.5 to 2 mg of the oestrogen component (II) and 1 to 10 mg of the gestagen component and optionally conventional auxiliary substances.

5. Use according to one of claims 1 to 4, **characterised in that** one of the following components a) to c) or h) is used as the gestagen component,
a) 3α-hydroxy-chlormadinone acetate or
b) 3β-hydroxy-chlormadinone acetate or
c) a mixture of a) and b) in any desired mixing ratio or
h) a mixture of chlormadinone acetate with a) and/or b) in a mixing ratio of 10 to 90% by wt. of CMA and 90 to 10% by wt. of a) and/or b), relative to the total mixture.

6. Use according to claim 1, **characterised in that** a daily unit is produced using a hormone combination consisting in each case of at least 15 µg, preferably 20 µg or 30 µg of ethinyl estradiol and/or at least 0.5 mg, preferably 1 mg or 2 mg of estradiol and in each case at least 1, preferably 2, 3, 4 or 5 mg of one of gestagen components a) to c) or h) and optionally conventional auxiliary substances.

7. Use according to claim 6, **characterised in that** the hormone combination consists in each case of 20 µg or 30 µg of ethinyl estradiol and/or 1 mg of estradiol and ≥ 2 mg of the gestagen components a) to c) or h).

8. Use according to one of claims 1 to 7, **characterised in that** the medicinal drug is provided in the form of at least 21 daily units containing the hormone combination for uninterrupted administration optionally followed by a 7 to 3 day intake break or by 7 to 3 hormone-free daily units for uninterrupted administration.

9. Use according to claim 8, **characterised in that** the medicinal drug is provided in the form of daily units containing the hormone combination for uninterrupted administration over several years, preferably for up to 2 years, particularly preferred up to 1 year, and of 7 to 3 hormone-free daily units for uninterrupted administration or followed by an intake break of 3 to 7 days.

10. Use according to claim 8, **characterised in that** the medicinal drug is provided in the form of 77 to 193 daily units containing the hormone combination for uninterrupted administration and of 7 to 3 hormone-free daily units for uninterrupted administration or followed by an intake break of 3 to 7 days.

11. Use according to claim 8, **characterised in that** the medicinal drug is provided in the form of 42 to 52 daily units containing the hormone combination for uninterrupted administration and of 7 to 3 hormone-tree daily units for uninterrupted administration or followed by an intake break of 3 to 7 days.

12. Use according to claim 8, **characterised in that** the medicinal drug is provided in the form of 21 to 25 daily units containing the hormone combination for uninterrupted administration and of 7 to 3 hormone-free daily units for uninterrupted administration or followed by an intake break of 3 to 7 days.

13. Use according to any one of claims 1 to 12, **characterised in that** in each of the daily units containing the hormone combination the medicinal drug comprises quantitatively the same combination of ethinyl estradiol or estradiol and a gestagen component a) to c) or h).

14. Use according to one of claims 1 to 13 for preventing menstrual cycle-dependent mood swings, preferably for preventing a deterioration in emotional state in women.

15. Use according to one of claims 1 to 13 for balancing menstrual cycle-dependent mood swings.

16. Use according to one of claims 1 to 13 for improving the emotional state and mood over an entire female menstrual cycle.

17. Use according to one of claims 7 to 13 for alleviating menstrual cycle-dependent mood swings in women.

## Revendications

1. Utilisation d'une association d'hormones constituée d'au moins un oestrogène choisi parmi l'éthinylestradiol (I) ou l'oestradiol (II) en tant que composant oestrogène et d'au moins un métabolite de l'acétate de chlormadinone choisi parmi la 3α-hydroxy-6-chloro-17α-acétoxy-4,6-prégnadién-20-one (acétate de 3α-hydroxy-chlormadinone), ou la 3β-hydroxy-6-chloro-17α-acétoxy-4,6-prégnadién-20-one (acétate de 3β-hydroxy-chlormadinone), éventuellement en mélange avec de l'acétate de chlormadinone en tant que composant progestatif, pour la fabrication d'un médicament destiné à atténuer les changements d'humeur dépendant du cycle menstruel.

2. Utilisation selon la revendication 1, **caractérisée en ce que** le médicament est présenté sous forme de doses unitaires journalières.

3. Utilisation selon la revendication 1 ou 2, **caractérisée en ce que** pour la fabrication d'une dose unitaire journalière on utilise une association d'hormones constituée de 5 à 50 µg du composant oestrogène (I) et/ou 0,5 à 4 mg du composant oestrogène (II) et de 1 à 10 mg du composant progestatif et éventuellement des adjuvants usuels.

4. Utilisation selon la revendication 1 ou 2, **caractérisée en ce que** pour la fabrication d'une dose unitaire journalière on utilise une association d'hormones constituée de 5 à 30 µg du composant cestrogène (I) et/ou 0,5 à 2 mg du composant oestrogène (II) et de 1 à 10 mg du composant progestatif et éventuellement des adjuvants usuels.

5. Utilisation selon l'une quelconque des revendications 1 à 4, **caractérisée en ce qu'**on utilise comme composant progestatif l'un des composants a) à c) ou h) suivants,
a) acétate de 3α-hydroxy-chlormadinone ou
b) acétate de 3β-hydroxy-chlormadinone ou
c) un mélange de a) et b) en un rapport de mélange quelconque ou
h) un mélange d'acétate de chlormadinone avec a) et/ou b) en un rapport de mélange de 10 à 90 % en poids de CMA et 90 à 10 % en poids de a) et/ou b), par rapport au mélange total.

6. Utilisation selon la revendication 1, **caractérisée en ce que** pour la fabrication d'une dose unitaire journalière on utilise une association d'hormones constituée de respectivement au moins 15 µg, de préférence 20 µg ou 30 µg d'éthinylestradiol et/ou au moins 0,5 mg, de préférence 1 mg ou 2 mg d'oestradiol et respectivement au moins 1, de préférence 2, 3, 4 ou 5 mg de l'un des composants progestatifs a) à c) ou h) et éventuellement des adjuvants usuels.

7. Utilisation selon la revendication 6, **caractérisée en ce que** l'association d'hormones consiste en respectivement 20 µg ou 30 µg d'éthinylestradiol et/ou 1 mg d'oestradiol et ≥ 2 mg du composant progestatif a) à c) ou h).

8. Utilisation selon l'une quelconque des revendications 1 à 7, **caractérisée en ce que** le médicament est présenté sous forme d'au moins 21 doses unitaires journalières contenant l'association d'hormones pour l'administration ininterrompue éventuellement suivie d'une pause de prise pendant 7 à 3 jours ou de 7 à 3 doses unitaires journalières sans hormones, pour l'administration ininterrompue.

9. Utilisation selon la revendication 8, **caractérisée en ce que** le médicament est présenté sous forme de doses unitaires journalières contenant l'association d'hormones pour une administration ininterrompue pendant plusieurs années, de préférence jusqu'à 2 ans, de façon particulièrement préférée jusqu'à 1 an, et de 7 à 3 doses unitaires journalières sans hormones pour l'administration ininterrompue ou suivies d'une pause de prise pendant 3 à 7 jours.

10. Utilisation selon la revendication 8, **caractérisée en ce que** le médicament est présenté sous forme de 77 à 193 doses unitaires journalières contenant l'association d'hormones pour l'administration ininterrompue et de 7 à 3 doses unitaires journalières sans hormones pour l'administration ininterrompue ou suivies d'une pause de prise pendant 3 à 7 jours.

11. Utilisation selon la revendication 8, **caractérisée en ce que** le médicament est présenté sous forme de 42 à 52 doses unitaires journalières contenant l'association d'hormones pour l'administration ininterrompue et de 7 à 3 doses unitaires journalières sans hormones pour l'administration ininterrompue ou suivies d'une pause de prise pendant 3 à 7 jours.

12. Utilisation selon la revendication 8, **caractérisée en ce que** le médicament est présenté sous forme de 21 à 25 doses unitaires journalières contenant l'association d'hormones pour l'administration ininterrompue et de 7 à 3 doses unitaires journalières sans hormones pour l'administration ininterrompue ou suivies d'une pause de prise pendant 3 à 7 jours.

13. Utilisation selon l'une quelconque des revendications 1 à 12, **caractérisée en ce que** le médicament présente dans chacune des doses unitaires contenant l'association d'hormones quantitativement la même association d'éthinylestradiol ou oestradiol et d'un composant progestatif a) à c) ou h).

14. Utilisation selon l'une quelconque des revendications 1 à 13 pour empêcher des changements d'humeur dépendant du cycle menstruel, de préférence pour empêcher une détérioration de l'état psychique chez la femme.

15. Utilisation selon l'une quelconque des revendications 1 à 13 pour l'atténuation des changements d'humeur dépendant du cycle menstruel.

16. Utilisation selon l'une quelconque des revendications 1 à 13 pour l'amélioration de l'état psychique et l'apaisement de l'humeur pendant la totalité d'un cycle menstruel féminin.

17. Utilisation selon l'une quelconque des revendications 7 à 13 pour l'atténuation des changements d'humeur dépendant du cycle menstruel chez la femme.
